Europäisches Patentamt

European Patent Office

Office européen des brevets

(19)

(11) Publication number: 0 337 625
A2

(12) **EUROPEAN PATENT APPLICATION**

(21) Application number: 89303015.5

(51) Int. Cl.⁴: **C12Q 1/68 , C12N 15/00**

(22) Date of filing: 28.03.89

(30) Priority: 13.04.88 GB 8808680
25.04.88 GB 8809753

(43) Date of publication of application:
**18.10.89 Bulletin 89/42**

(84) Designated Contracting States:
**AT BE CH DE ES FR GB GR IT LI LU NL SE**

(71) Applicant: **IMPERIAL CHEMICAL INDUSTRIES PLC**
**Imperial Chemical House Millbank**
**London SW1P 3JF(GB)**

(72) Inventor: **Barnes, Stephen Robert**
**416 Reading Road Winnersh**
**Wokingham Berkshire, RG11 5EP(GB)**
Inventor: **Chojecki, Anthony John Stefan**
**79 Sinclair Road**
**London, W14(GB)**
Inventor: **Daly, Allan**
**159 Fernbank Road**
**North Ascot Berkshire, SL5 8JU(GB)**

(74) Representative: **Roberts, Timothy Wace et al**
**Imperial Chemical Industries PLC Legal**
**Department : Patents PO Box 6 Bessemer**
**Road**
**Welwyn Garden City Herts, AL7 1HD(GB)**

(54) **Probes.**

(57) Process for producing plant hypervariable DNA probes for characterising plants by using Jeffreys probes to isolate homologous sequences in plants, which are then cloned for use as probes. Also claims the probes so obtained, and their sequences, as well as their use to characterise plant material.

EP 0 337 625 A2

## PROBES

This invention relates to probes, and more particularly to hypervariable DNA probes useful in characterising living material.

As is well known, the genome of an organism comprises one or more chains of DNA. These chains contain the genes from which are made the proteins which carry out the functions of the living cell. Associated with the genes are upstream and downstream control areas which may, for example, serve to switch the gene on and off. The function of such DNA, involved in genes and control sequences, is at least in principle understood. However, it makes up quite a small proportion of the total DNA to be found in the genomes of higher organisms. In most organisms, most of the DNA does not form part of genes or control sequences. Its function is not understood, and indeed it may not have any important biological function. It may simply be reproduced, from generation to generation, as a photocopier will reproduce a meaningless blemish on the paper of an original document. Some of this possibly functionless DNA consists of the same short base sequence many times repeated. Having (apparently) no biological function at the cell level, such regions of repeated sequences are highly variable in length, constitution, and position in the genome. Herein they are termed "hypervariable" sequences.

Two major types of tandemly repeated sequences are recognised: "satellite" and "minisatellite" DNA. Satellite DNA is encountered in certain chromosomal regions, for example near centromeres and telomeres, and typically consists of $10^3$ - $10^5$ sequence repeats at each site. Minisatellite DNA has been found in vertebrate genomes, and comprises fewer repeats (generally of the order of tens or hundreds) at each site: sites are often widely dispersed throughout the chromosomes.

The number of repeats at a particular chromosome locus often varies. This results in many different allelic forms at each locus. As there are many such loci, together they form a set of genomic regions that, taken together, vary widely between unrelated individuals. Hence the term 'hypervariable'.

In Patent 2166445B (patentee Lister Institute of Preventive Medicine) Dr A J Jeffreys discloses a method of using specified DNA probes derived from hypervariable minisatellite regions of DNA to provide genetic fingerprints. The probes are used in Southern blot analysis. Probes based on such minisatellites hybridise to test DNA at many different points, because of the many different places at which such regions oGCur within the genome. Thus, if a sample of unknown DNA is digested with a suitable restriction enzyme, size sorted by electrophoresis and hybridised with a minisatellite probe under the standard conditions of the Southern test, a very detailed picture containing many bands is obtained. The structure of the genome determines the banding pattern: identical genomes give identical banding patterns. Closely related genomes give patterns which are similar but different, allowing degrees of relatedness to be assessed.

The method described by Jeffreys in the above-mentioned UK Patent 2166445B (the disclosure of which is herein incorporated by reference) has been used very suGCessfully in demonstrating similarity and difference between related individuals (animals as well as humans) on the basis of tests on their DNA. In particular, it has been used forensically to prove the identity of criminals from traces left behind at the scene of the crime: and in cases of disputed parentage (for example in immigration disputes).

UK Patent 2166445 foresees that the Jeffreys probes may have application to plant breeding, as well as human and animal investigations where they have hitherto mainly been used. However, our own investigations in this area, using Jeffreys probes to produce genetic fingerprints of plant material, have given a high level of background hybridisation to a smear of fragments, over which faint bands are discernible. The patterns obtained though varying from species to species are generally less clear and intense than is suitable for wide practical use.

We have now discovered a way of building on the technology disclosed in UK Patent 2166445 to provide probes which have wider applicability in plants, giving clear autoradiographs with short exposure times, enabling distinctions to be made between distinct but related plant lines. Thus, aGCording to the present invention we provide a process for obtaining hypervariable DNA probes useful in characterising plant material which comprises: preparing purified DNA from a target plant species; cutting the purified DNA into fragments; cloning the fragments; screening the cloned fragments with a Jeffreys probe (as hereinafter defined) and selecting one or more clones binding to said probes; and multiplying such selected clones.

In a further aspect our invention provides hypervariable DNA clones useful in the characterisation of plant material and obtained by the process defined above.

Our invention also comprises hypervariable DNA sequences useful in the characterisation of plant material, and having the repeat tandem sequence: AGAGGGCGGCGG. Our invention further comprises sequences having a high degree of homology therewith (eg. 70 or 80%).

Our invention further comprises sequences having a degree of homology with such sequences

EP 0 337 625 A2

sufficient to hybridise thereto.

Two DNA sequences are said to have 100% homology when each base in one sequence matches the corresponding base in the other sequence. If the match is incomplete, homology is less than 100%. For the purpose of the present invention, we measure homology between two DNA sequences by comparing two similar sequences at least one of which is eighteen bases long. Percent homology is then defined as the minimum number of changes (base changes, deletions or additions) per 100 bases required to make one sequence identical with the other.

We find that the sequences will generally hybridise if they show at least 70%, preferably 80% homology (as defined above), and aGCordingly our invention further comprises clones having at least 70%, and preferably 80% homology (as above defined) to repeated AGAGGGCGGCGG sequences.

We are not certain whether the hypervariable sequences of the present invention have the same origin or function as previously known hypervariable sequences; for the purposes of our invention its is enough that they appear to behave in a similar way.

Our invention is to be applied throughout the plant kingdom. It is particularly useful in connection with crop plants, which may be either monocotyledonous or dicotyledonous. Examples of monocotyledonous crops are rice, wheat, barley, rye, oats, Allium species, eg onions; forage grass species, Triticale, sorghum, millet and (in particular) maize (Zea mays). Examples of dicotyledonous crops are sugar beet, tomatoes, tobaGCo, lettuce, peas, beans, alfalfa, sunflower, soya and potatoes, and Brassica species such as cabbage, rape and Brussels sprouts.

Our invention is also applicable to trees and bushes, for example forestry species such as pines, firs, oak, ash, beech, sycamore; nuts; plantation crops, eg. rubber and oil palm; fruit species including fruit trees and bushes such as apple, pear, plum, cherry, orange, lemon, banana, blackcurrant, gooseberry, peach, raspberry as well as fruit plants such as strawberry; and ornamentals, eg, roses.

The probes used by Jeffreys are defined in claim 1 of UK Patent 2166445 as polynucleotides in isolated or cloned form having the general formula, read in the $5'$ to $3'$ sense

H (J core K)$_n$.L     (1)

wherein:

"core" represents a sequence having at least 6 consecutive nucleotides, selected from within any of the following sequences read in the same sense.

GGAGGTGGGCAGGAXG     (2)
AGAGGTGGGCAGGTGG     (3)
GGAGGYGGGCAGGAGG     (4)
T(C)$_m$GGAGGAXGG(G)$_p$C     (5A)
T(C)$_m$GGAGGA(A)$_q$GGGC

(5B)

wherein:

(i) X is A or 6, Y is C or T, T = T or U, m is 0, 1 or 2, p is 0 or 1, q is 0 or 1, n is at least 3;

(ii) J and K together represent 0 to 15 additional nucleotides within the repeating unit; and

(iii) H and L each represent 0 or at least 1 additional nucleotide flanking the repeating units,

(iv) the repeating unit (J Core K) need not be an exact repeat either as to number and kind of nucleotides, provided that the polynucleotide has a recognisably repeated consensus sequence,

(v) "core" can also represent a variant core sequence, provided that actual core sequences in all n repeating units have on average at least 70% homology with "true" core sequences as defined above with respect to formulae 2 to 4, 5A and 5B

or a polynucleotide of complementary sequence to the above, the polynucleotide being capable of hybridising with fragments of DNA from more than one minisatellite region or hypervariable locus produced by one or more restriction enzymes which do not cleave to any relevant extent within a sequence corresponding to the repeating unit (J core K).

AGCordingly, in the present specification by the term "Jeffreys probe" we mean a probe as defined above, or as otherwise disclosed or claimed in UK Patent 2166445B.

Our invention further comprises a process for characterising plant material which comprises preparing purified DNA from test material: cutting the purified DNA with a selected restriction enzyme; separating the cut DNA into an array of fragments by a standard separation technique; probing the array of fragments with a hypervariable probe produced aGCording to the process of the invention; visualising the probe fragment hybridisations and comparing the pattern so obtained with a corresponding pattern obtained from characterised plant material using the same restriction enzyme and the same probe under the same conditions. Generally, the comparison enables one to infer a degree of identity or similarity between the test and characterised plant materials corresponding to the identity and similarity of the two patterns. The compari-

3

son may also indicate whether the test material is heterozygous or homozygous.

We find that particularly good results may be obtained by using hypervariable probes derived from the same plant genus as the test plant material. For example, hypervariable probes derived from maize may be used to characterise unknown maize plants and compare them with known lines: the same may be done with rice. In a further aspect our invention comprises a process for obtaining hypervariable DNA probes useful in characterising plant material analogous to that described above, but using, in place of a Jeffreys probe, a probe obtained by the process of the present invention.

There are many potential applications of probes of the present invention in plant genetic analysis. They have immediate value for use in genetic fingerprinting, in the context of plant variety description for registration and like industrial property purposes. They can also produce information of practical value to plant breeders. For example, the fingerprints obtainable may be used to determine the pedigree of a plant variety, by comparison with fingerprints of other possible parent lines. The genetic composition of a hybrid may be determined similarly, to ensure that hybrid seed lots are pure hybrid, free of parental pure line seed and other contaminating seed. Conversely, the products and methods of the invention can assist in the production of pure line varieties or inbred parent lines by identifying homozygous individuals on a breeding programme, and ensuring genetic uniformity of seed lots. Such tasks may in principle be performed by known techniques using morphological markers, isozyme analysis or even single locus RFLP (restriction fragment length polymorphisms) technology, but the methods of the present invention are very much quicker, simpler, more reliable and hence generally more practicable.

Aspects of the invention will be further described with reference to the aGCompanying drawings, in which:

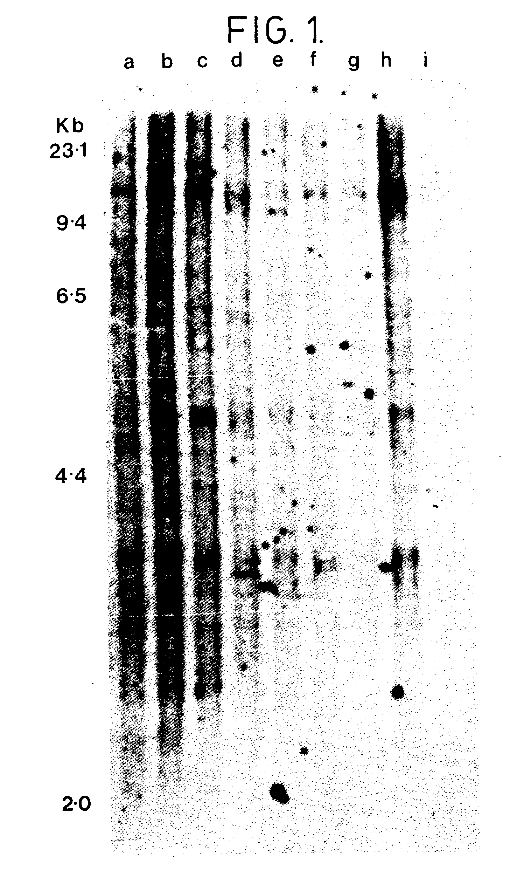

Figure 1 is a Southern blot of genomic DNA of different maize varieties probed with the Jeffreys 33.6 human hypervariable probe. The maize genomic DNA is digested with DraI. Lanes: a - PE38; b - RW01; c - GR39; d - S062; e - CV32; f - CB58; g - AM62; h - P7; i - BE70.

Figure 2 is a Southern blot analysis of rice clones aGCording to the invention selected using the human minisatellite probe 33.15. Four restriction enzymes were used on each of five clones. (a - HinfI; b - HaeIII; c -DraI; d - HindIII).

Figure 3 is a Southern blot of genomic DNA of different rice varieties probed with a fragment of the rice clone 15.2/2 of the invention. Rice DNA was digested with DraI (lanes a-h) and EcoR1 (lanes i-p). Lanes: a,i - IR36; b,j - IR45; c,k - Taipei 309; d,l - 056; e,m - Silewah; f,n - Fujisaka 65; g,o - Yamassiko; h,p - Taichung 65.

Figure 4 is a Southern blot of genomic DNA of different rice varieties probed with a fragment of the rice clone of the invention 15.2/2. Rice DNA was digested with BamH1 (lanes a-h) and HindIII (lanes i-p). Lane: a,i -Taichung 65; b,j - Yamassiko; c,k - Fujisaka 65; d,l -Silewah; e,m - 056; f,n - Taipei 309; g,o - IR45; h,p -IR36.

Figure 5 is a Southern blot of genomic DNA of different rice varieties probed with a fragment of the rice clone 15.4/2 of the invention. Rice DNA was digested with DraI. Lanes: a - IR45; b - Fujisaka 65; c - Silewah; d -Yamassiko; e - Taichung 65; f - 065; g - Taipei 309; h -IR36.

Figure 6 is a Southern blot of genomic DNA of different rice varieties probed with a fragment of the rice clone 15.7/1 of the invention. Rice DNA was digested with DraI. Lanes: a - IR36; b - Taipei 309; c - 056; d -Taichung 65; e - Yamassiko; f - Silewah; g - Fujisaka 65; h - IR45.

Figure 7 is a Southern blot of genomic DNA of different rice varieties probed with a fragment of the rice clone 15.7/1 of the invention. Rice DNA was digested with BamH1 (lanes a-h) and HindIII (lanes i-p). Lanes: a,i -IR36; b,j - IR45; c,k - Taipei 309; d,l - 056; e,m -Silewah; f,n - Fujisaka 65; g,o - Yamassiko; h,p - Taichung 65.

Figure 8 is a digitised version of Figure 3: lanes 2-17 correspond to lanes a-p in Figure 3.

Figure 9 gives a partial nucleotide sequence of the rice 15.2/2 BamH1 fragment.

Figure 10 gives the nucleotide sequence of the maize 33.6c BamH1 fragment.

Figure 11 compares schematically the rice and maize genomic clones 15.2/2 and 33.6c.

Figure 12 is a listing comparing the amino acid sequence encoded by the open reading frames in the rice and maize clones 15.2/2 and 33.6c

Figure 13 shows two Southern blots of genomic rice DNA from 5 different inbred lines (a - e) digested with EcoRI and BamH1 and probed with E15.2f5d (A) and AN1 (B). Lanes: a - Silewah; b - 056; c - T909; d - IR45; e - IR36.

Figure 14 is a Southern blot of genomic rice DNA from 3 different inbred lines (a - c) digested with 6 different restriction enzymes and probed with AN1. Lanes: Control -linearised M13 containing the AN1 clone; a - Silewah; b -T909; c - 056.

Figure 15 is a Southern blot of genomic rice DNA from 3 different inbred lines (a - c) digested with 6 different restriction enzymes and probed with E15.2f5d. Lanes: Control - linearised M13 containing the E15.2f5d clone; a -Silewah; b - T309; c - 056.

Figure 16 is a Southern blot of genomic rice DNA from 7- different inbred lines (P1 and P3 - P8) digested with restriction enzyme BamH1 and probed with AN1. Lanes: P1-IR36; P3 - IR39334; P4 - TN1; P5 - IR39357; P6 - IR39385; P7 - IR39422; P8 - Kwang Lu Ai 4.

(1) Cloning

Partial Sau 3A digests of genomic DNA from Oryza sativa var Indica TNI and of Zea mays var.BE10 were size fractionated on neutral sucrose gradients [Maniatis, Hardison, Lacy, Lauer, O'Connell, Quon, Sim and Efstratiadis (1978) Cell 15, 687]. Fragments in the 15-20 kb size range were ligated to the BamH1 HI sites of left and right arms of bacteriophage lambda EMBL3, obtained in purified form from Amersham International. Recombinant bacteriophage were in vitro packaged (Amersham kit) and libraries constructed by infecting E. coli strain NM621. A portion of each library was screened by hybridisation with Jeffreys probes 33.15 and 33.6 using the plaque-lift procedure [Maniatis, Fritsch and Sambrook (1982) Molecular Cloning, CSHL Press]. Positive clones were identified at frequencies shown in Table I.

(2) Isolation of plant minisatellite sequences

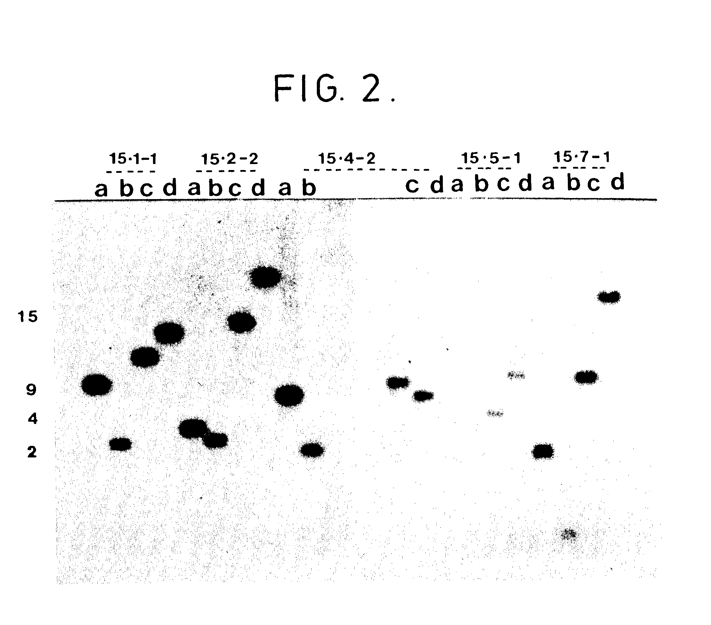

Five of the positive rice lambda clones and four of the maize clones were analysed (Table 2). The original positive plaques were purified by reinfecting E. coli NM 621, replating at low density and rescreening using the human minisatellite probes. Recombinant bacteriophage DNA was prepared by the method of Yamamoto, Alberts, Benzinger, Lawhorne and Treiber [Virology 40, 734 (1970)] followed by equilibrium centrifugation of the PEG precipitate in ceasium chloride gradients. Approximately 1 μg of each phage DNA was analysed by Southern blot hybridisation [Southern (1975) J. Mol. Biol. 93, 503] using the human probes to identify the fragments containing sequences homologous to the human minisatellite sequence (Figure 2). These DNA fragments were then purified by isolation from low melting point agarose gels [Weislander (1979) Analytical Biochem 98, 305] and either used as probes or subcloned for DNA sequence analysis.

(3) Southern blot hybridisation using plant probes.

Genomic DNA from different species, including rice, maize, wheat and barley was digested with various restriction enzymes, electrophoresed in 0.8% agarose gels and transferred to nylon membranes using alkaline blotting conditions (Zetaprobe). Cloned rice sequences isolated as in (2) above were labelled with 32P using the random oligonucleotide priming method [Feinberg and Vogelstein (1984) 137, 266]. Filters were prehybridised for 6 hours in one of two mixtures: (A) 5x SSC(sodium chloride/citrate), 50% formamide, 1% Cadbury's 'Marvel' powdered milk, 0.02% sodium azide and 1/40 M Na phosphate pH 6.5 at 42° C: or (B) 2x SSC, 50% formamide, 1% Cadbury's 'Marvel' powdered milk, 0.02% sodium azide and 1/40 M sodium phosphate (pH 6.5) at 65° C.

Hybridisation was either in mixture A at 42° C, or in mixture B at 65° C, plus 10% dextran sulphate and labelled probe (40 ng DNA/ml, specific activity 2.5 x 10⁸ cpm/μg) for 16 hours. Filters were washed for 2 x 30 minutes with 2x SSC, 0.1% SDS at 65° C, and once for 30 minutes in 0.1x SSC, 0.1% SDS (sodium dodecylsulphate) at 65° C. Filters were autoradiographed at -70° C for 2-24 hours.

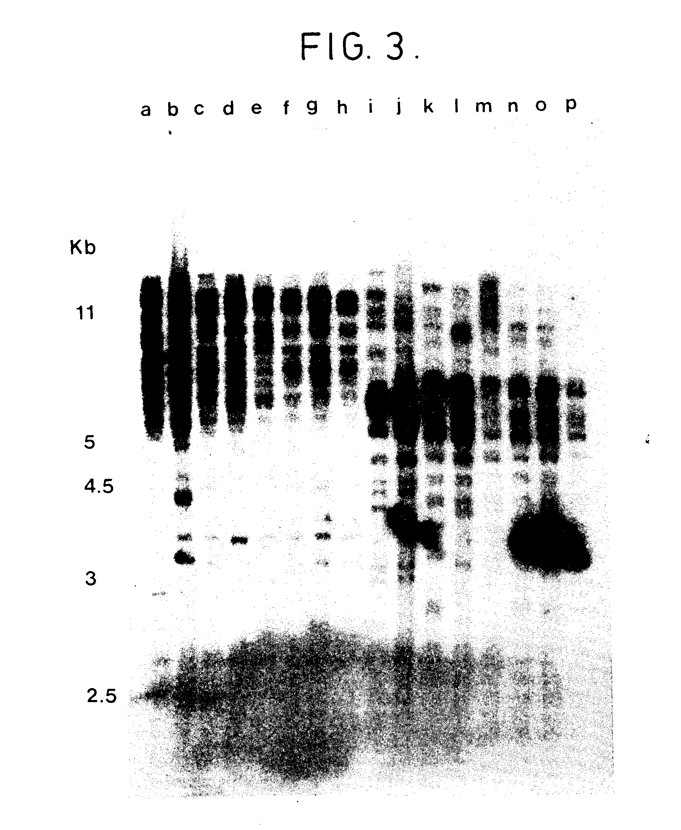

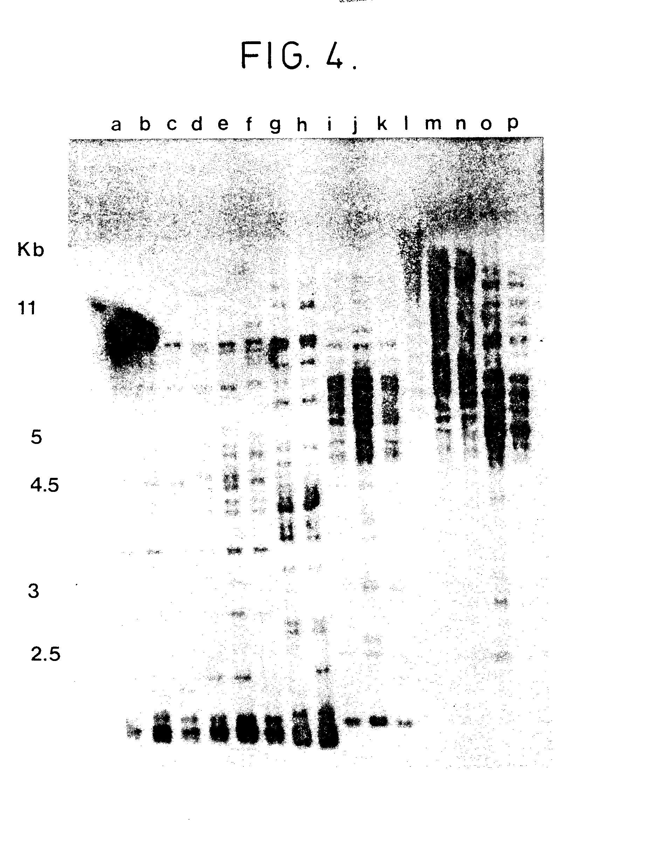

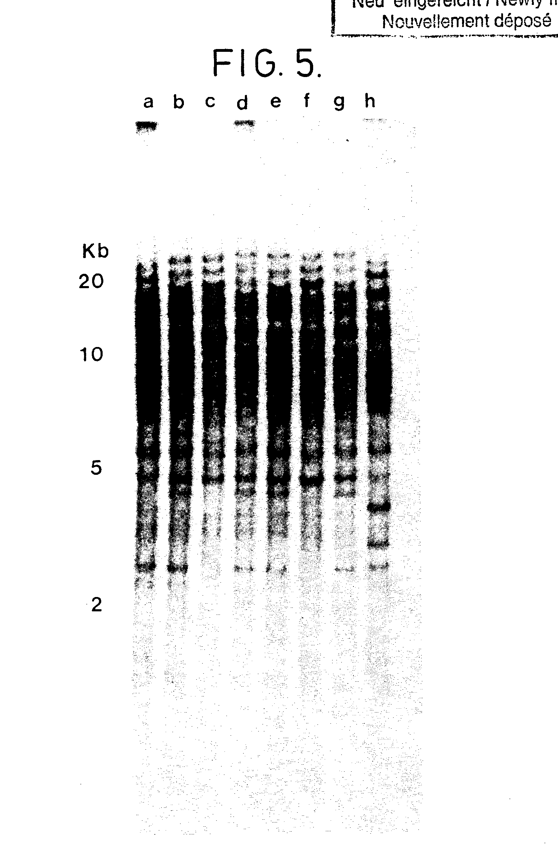

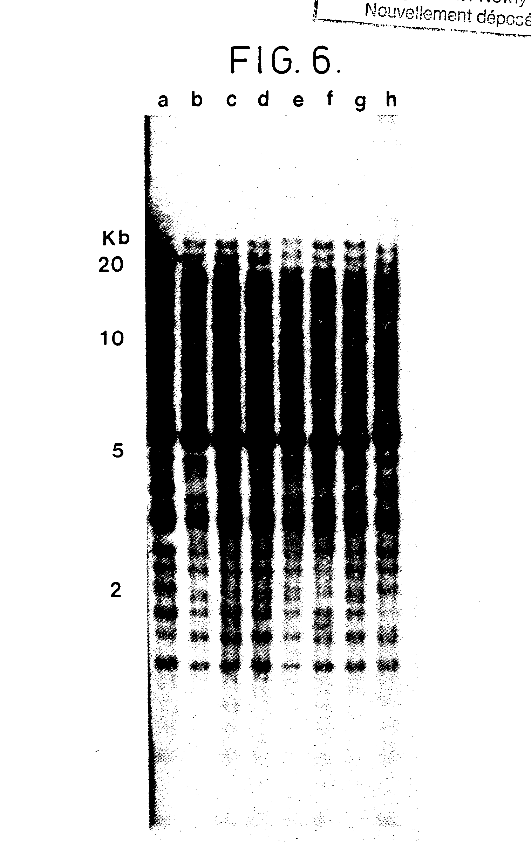

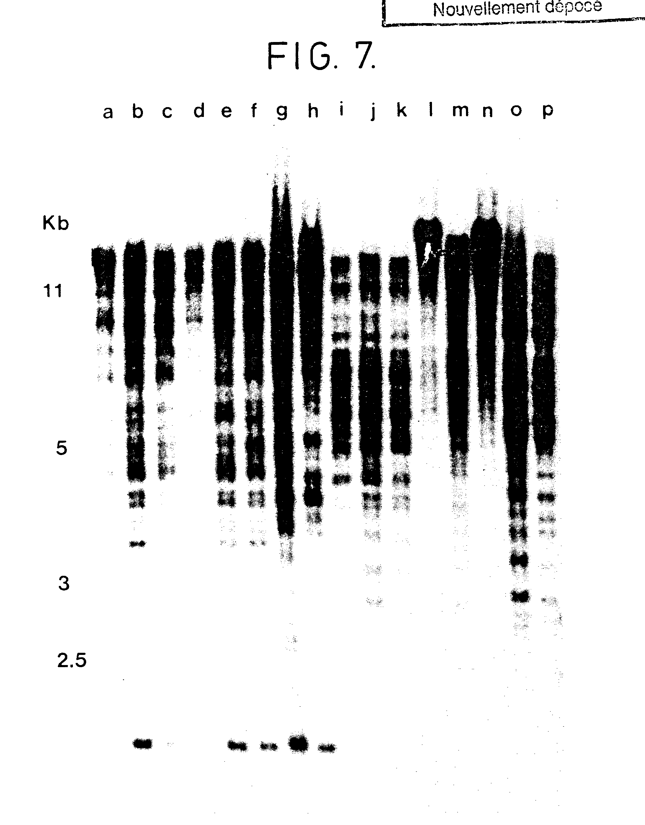

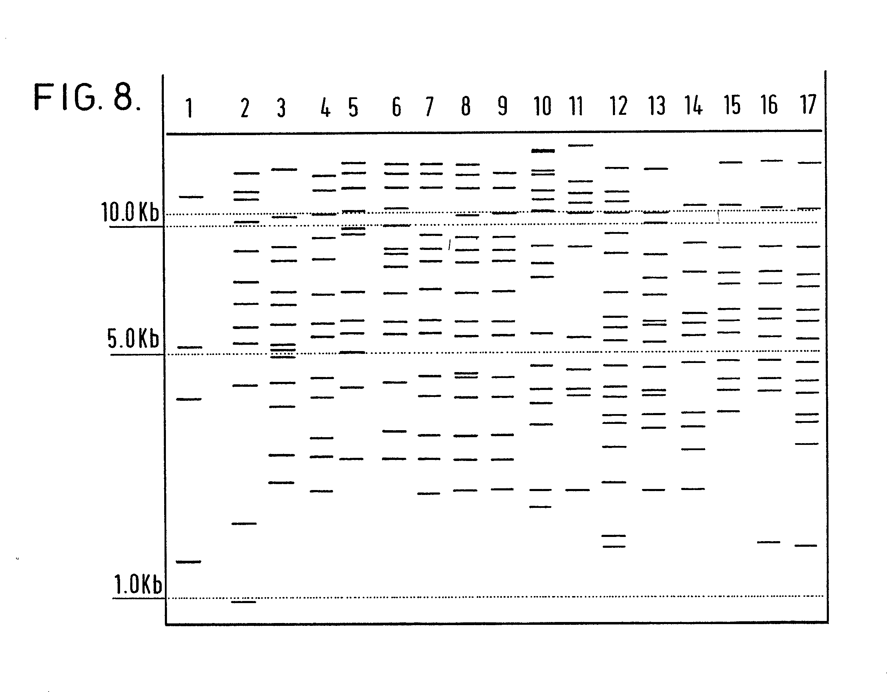

The rice probes hybridise very strongly to rice genomic DNA giving clear signals with overnight exposures (Figures 3-8). Polymorphic patterns, similar to those seen with human hypervariable probes in vertebrate studies, were obtained when a range of different rice cultivars were compared. The different rice probes (including those identified with the same original human probe) give different fingerprints from each other.

(4) Sequence analysis of plant hypervariable DNA.

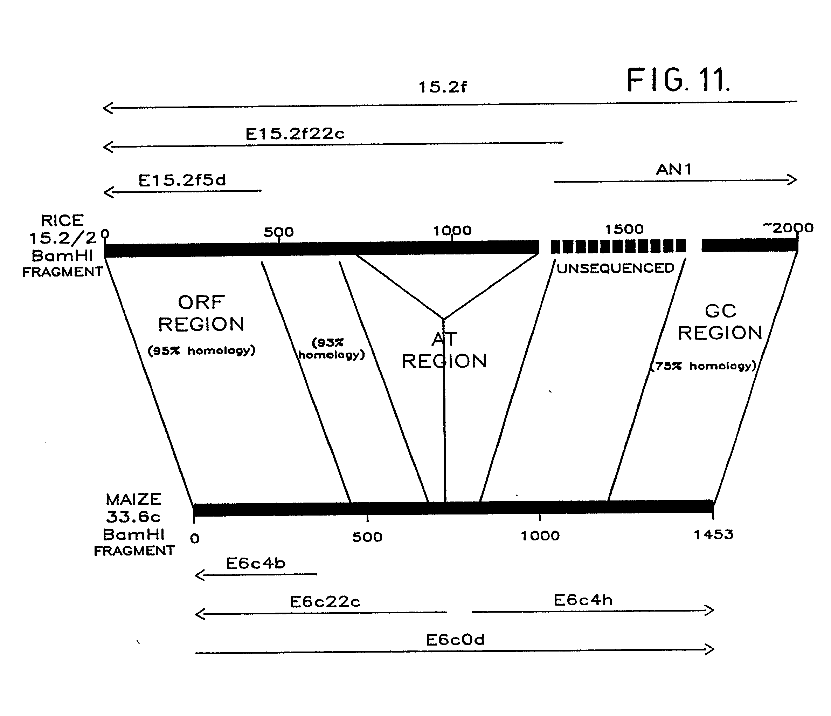

Fragments isolated as in (2), above, were subcloned into the sequencing vector M13mp18 and sequenced by the method of Sanger et al (Proc Natl Acad Sci USA 74,5463-5467, (1977)). Further sequence information was obtained from nested series of fragments produced by exonuclease III digestion (Henikoff,S. Gene 28, 351 (1984) ). Two such fragments are E15.2f5d and AN1. These are both obtained from rice clone 15.2/2 (see Table 2). These and other fragments are shown schematically in Figure 11.

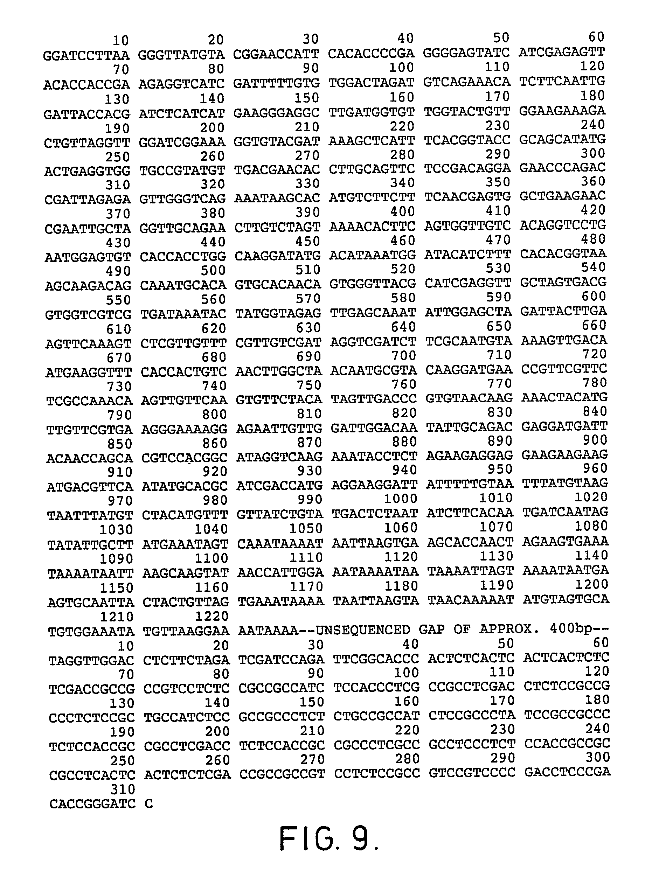

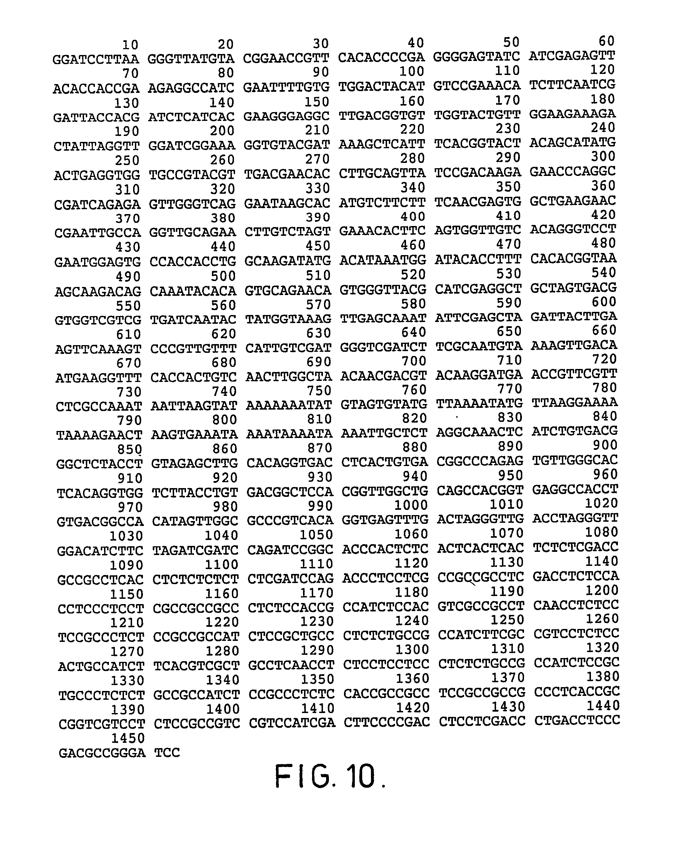

Figures 9 and 10 show representative sequences of cloned rice and maize genomic sequences that were isolated aGCording to the method of the invention. The sequences shown are of the rice clone 15.2/2, in Figure 9, and the maize clone 33.6c (see Table 2) in Figure 10. Figure 11 shows the relationship between these probes and several fragments derived from them.

Both 15.2/2 and 33.6c clones contain GC-rich regions, with some homology to the Jeffreys probes used to identify them. Table 3 shows that these sequences may be re-written as a series of consecutive 12bp repeats, having a consensus of AGAGGGCGGCGG, reading in the 5′ to 3′ direction. The sequence is also clearly based on a simpler, presumably ancestral, 3bp repeat unit. (Table 3 shows the rice sequence - in the opposite sense to that shown in Figure 9 - to permit easier comparison with the human and bacteriophage sequences to which they show some homology.) The tandem repeating structure revealed in this analysis shows the GC-rich region to be arranged in a typical "minisatellite" organisation.

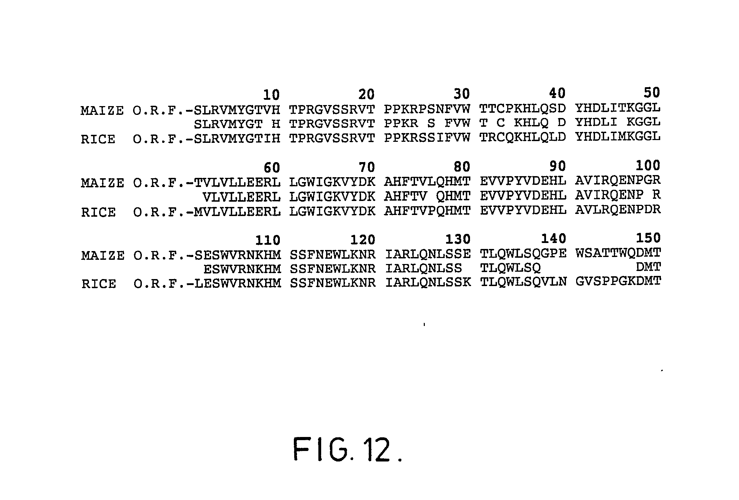

The GC-rich repeating units in the clones of our invention (hereafter referred to as GC or GC repeats) are (in all cases so far examined) downstream of the 3′ end of an open reading frame (ORF)(by downstream, we mean at the 3′ end of the coding-sequence, on the coding strand of the DNA; thus in the direction in which a gene sequence is transcribed by RNA polymerase these sequences come after the gene on the coding strand). The ORF is virtually identical between clones for which full sequence information is available. It ends at bases 454-456 of the sequences shown in Figures 9 and 10; it begins before these sequences start (exactly where is not yet known). A comparison of the protein coding regions for the maize and rice clones is shown in Figure 12. In this Figure the letters represent amino-acid residues, using the conventional one-letter abbreviations.

In both species the region between ORF and GC repeats contains both unique (ie not repetitive within the cloned region) and AT-rich repeated sequences. The repeating AT-rich motif is stronger in rice (shown in Table 4) than in maize. The function (if any) of this region is not known, but we might expect it to be involved in the regulation of expression of the ORF regions.

## (5) Variability between individuals resides in DNA fragments containing the GC repeats

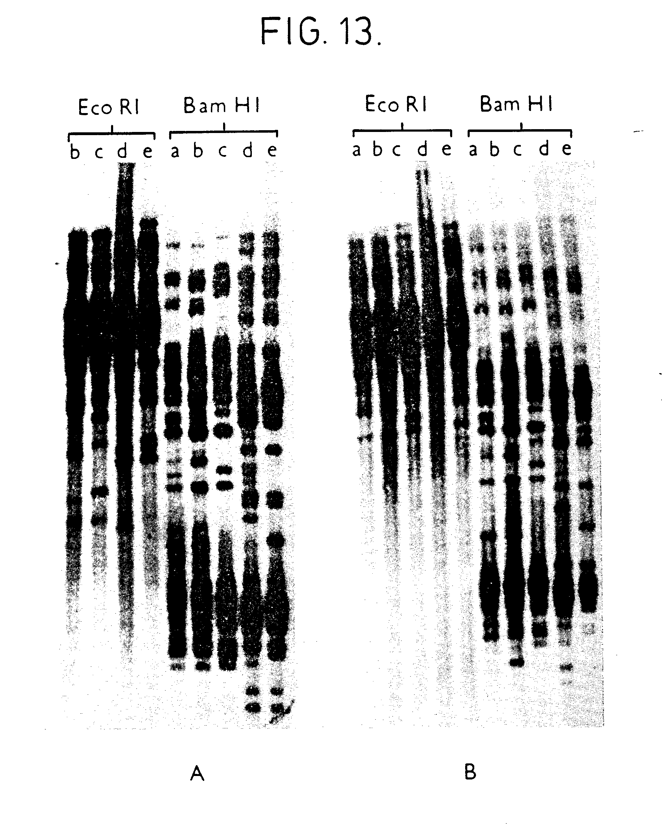

Rice genomic DNA from five different varieties was digested with restriction enzymes (Bam HI and Eco RI) that recognise sites not found between the GC and ORF regions. The digests were subjected to electrophoresis and Southern transfer, and probed with clones E15.2f5d and AN1. The results obtained are shown in Figure 13. These two clones represent the GC and ORF regions, respectively (see Figure 11). Using these restriction enzymes, both probes hybridise to essentially the same set of fragments.

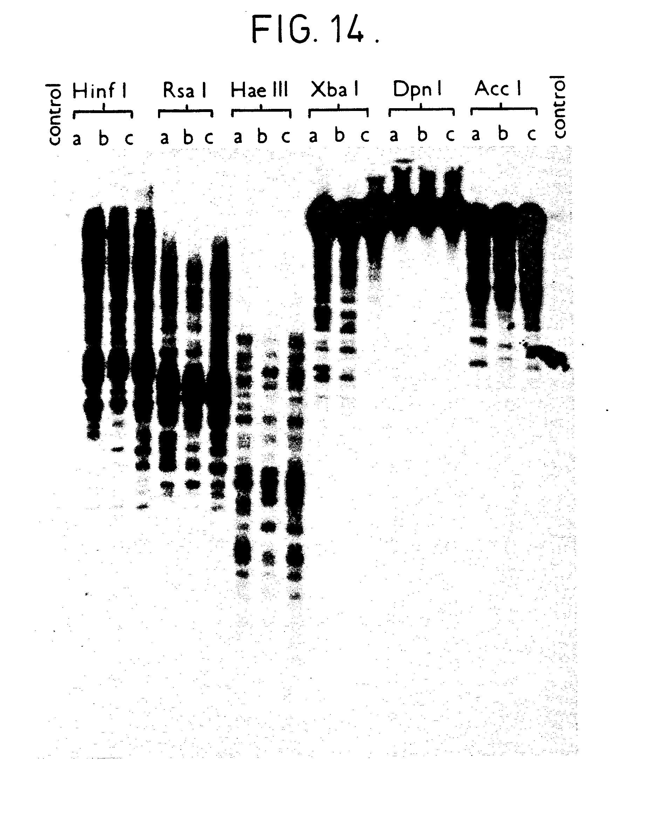

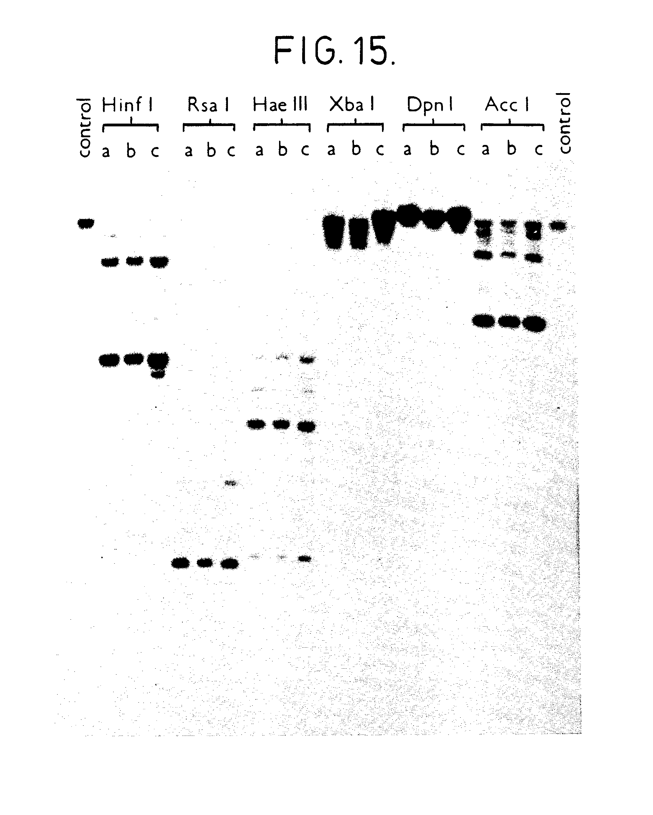

Certain enzymes, however, are capable of separating some, or the majority, of ORF sequences from their neighbouring GC repeats. When these enzymes are used for Southern analysis of genomic DNAs, it is the genomic fragments homologous to the GC probe that show variability between genotypes. This is illustrated by the experiments leading to Figures 14 and 15.

Genomic rice DNA from 3 different inbred lines (a -c) was digested with 6 different restriction enzymes (Hinf I, Rsa I, Hae III, Xba I, Dpn I and AccI) and probed with clone AN1. Figure 14 shows the resulting Southern blots. Lanes: Control - linearised M13 containing the AN1 clone; a - Silewah; b - T309; c - 056.

Genomic rice DNA from 3 different inbred lines (a -c) was digested with the same 6 restriction enzymes and probed with clone E15.2f5d. Figure 15 shows the resulting Southern blots. Lanes: Control - linearised M13 containing the E15.2f5d clone; a - Silewah; b - T309; c - 056.

The fragments containing the ORF (clone E15.2fd5) are relatively invariant (Figure 15). We interpret this result as showing that the pattern variability between lines is due to variation in or close to the GC region. Thus any restriction fragment containing this region may show useful variation, and any probe homologous to such a fragment may be used in Southern analysis to reveal the variation. Depending upon the probe selected, the variation will be detected at all loci, or at an individual locus (providing a single-locus probe).

The probes of the present invention may also be used to isolate locus-specific probes.

Locus-specific probes may be isolated aGCording to our invention by using the probes of our invention to identify specific plant DNA fragments, and cloning such fragments. The selection of such fragments so as to give useful locus-specific clones is largely a matter of trial and error, but suGCess is sufficiently frequent to make the technique a useful one. Generally fragments should be selected which give fainter Southern bands, indicating that they are less strongly hybridised and thus have a structure differing somewhat from that of the probe. Locus-specific probes should be used under conditions of high stringency, so that they bind only to closely similar sequences. Further information about locus-specific probes is given in EP 238329 (A2) (ICI).

The use of locus-specific hypervariable probes in the analysis of segregating populations will enable estimates to be made of linkage between molecular markers in the production of a genetic map, and correlations to be detected between hybridisation patterns and traits of agronomic importance. The probes may then have predictive value for plant breeding germplasm, similar to the diagnostic value of DNA probes for hereditary diseases in humans. Such probes would assist the transfer of specific chromosomal segments between breeding populations, or the detection of genetic diversity in new populations or alien species and its subsequent introduction into plant breeders' stock germplasm.

## (6) The use of plant minisatellite sequences to analyse plant lines

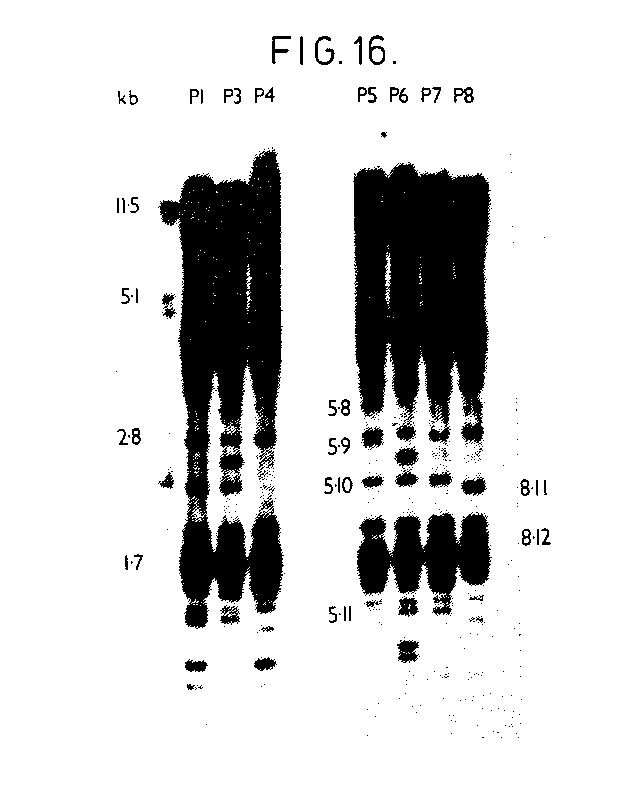

Genomic rice DNA from 7 different inbred lines (P1 and P3 - P8) was digested with Bam H1 and probed with AN1. Figure 16 shows the pattern resulting. Lanes: P1- IR36; P3 - IR39334; P4 - TN1; P5 - IR39357; P6 - IR39385; P7 -IR39422; P8 - Kwang Lu Ai 4. Table 5 gives the molecular sizes corresponding to some of the bands of hybridisation resolved on this autoradiogram.

Different electrophoresis conditions may be used to resolve bands migrating in the various molecular weight ranges. A number of bands are present in all lines; however, each lines has a characteristic set of additional bands that provide it with an individual "fingerprint".

By analysis of suitable autoradiograms, it is possible to resolve up to 30 bands with probe AN1, of which 10-15 show variation between pairs of lines.

The genomic (or chromosomal) distribution of sequence corresponding to these bands may be determined by classical genetic analysis. Linkage, either between bands revealed using this probe, or between these bands and other genetic markers (morphological or molecular, or characters of ecomonic importance) may be detected by examining the segregating progeny resulting from a cross between individuals with differing banding patterns. Table 6 shows the results of such an analysis of six varying bands in twenty F2 rice plans derived from a cross between lines P5 and P8 shown in Figure 16. Analysis of the segregation patterns reveals that:

a The bands from each parent are not inherited as a block.

b Many pairs of bands appear to be inherited independently of one another - ie they are not tightly linked.

The general conclusion from this and other genetic analyses is that the plant sequences corresponding to the probes of the invention are distributed over a dispersed set of genomic sites.

The clones in Table 2 have been deposited at the National Collections of Industrial and Marine Bacteria (NCIB), Torry Research Station, PO Box 31, 135 Abbey Road, Aberdeen AB9 8DB, Scotland. The corresponding NCIB numbers are listed in Table 2. E Coli strain NM 621 (ex Dr N E Murray, Edinburgh University) has also been deposited at NCIB under the number NCIB 40010.

TABLE 1

| Species | 33.15 | | | 33.6 | | |
|---|---|---|---|---|---|---|
| | Recombinant plaques | Positive plaques | Frequency | Recombinant plaques screened | Positive plaques | Frequency screened |
| O. sativa | $10^4$ | 8 | $8 \times 10^{-4}$ | $10^4$ | 2 | $2 \times 10^{-4}$ |
| Z. mays | $10^4$ | 5 | $5 \times 10^{-4}$ | $10^4$ | 4 | $4 \times 10^{-4}$ |

TABLE 2

| Clone | Plant DNA | Homologous human probe | Restriction enzyme to release Probe | Size of probe fragment (kb) | NCIB Deposit Number |
|---|---|---|---|---|---|
| 15.1/1 | rice | 33.15 | HaeIII | | 4001 |
| 15.2/2 | rice | 33.15 | BamHI | 2kb | 4002 |
| 15.4/2 | rice | 33.15 | BamHI | 2kb | 4003 |
| 15.5/1 | rice | 33.15 | | | 4004 |
| 15.7/1 | rice | 33.15 | PstI | 4kb | 4005 |
| 33.6B | maize | 33.6 | HaeIII | | 4006 |
| 33.6C | maize | 33.6 | HaeIII | | 4007 |
| 33.15D | maize | 33.15 | HaeIII | | 4008 |
| 33.15E | maize | 33.15 | HaeIII | | 4009 |

TABLE 3

```
A G A G G A T C C C G G        T

                      T
                      ∇
G T C G G G A G G C G G
        C
        ∇
G G A G G A C G G G G G        A G A
G G A C G G C G G C G G        T C G
A G A G A G T G A G T G
A G G C G G C G G C G G        T G G
A G A G G G A G G C G G
C G A G G G C G G C G G        T G G
A G A G G T C G - - - -
A G G C G G C G G T G G
A G A G G G C G G C G G
A T A G G G C G G - - -
A G A T G G C G G C A G
A G A G G G C G G C G G
A G A T G G C A G C G G
A G A G G G C G G C G G
A G A G G T C G A G C -
```

TABLE 4

```
                                    1039
                                     |
                       GTcAAATAAAATAATTAA
                       GTGAAgcAccAaCtagAA
                       GTGAAATAAAATAATTAA
                       GcaAgAatAAccAt
                       tgGAAATAAAATAATaAA
                       attAgTAAAATAAgTgA  A
                       GTGcAATtActactgTtA
                       GTGAAATAAAATAATTAA
                                             |
                                           1177
```

| | |
|---|---|
| G | 615012000000002110 |
| A | 011676167662651157 |
| T | 151100620015034510 |
| C. | 011100101211200000 |

Consensus     **GTGAAATAAAATAATTAA**

TABLE 5

| Molecular Weight of band (bp) | P1 | P3 | P4 | P5 | P6 | P7 | P8 | No of Parental Lines With the Band |
|---|---|---|---|---|---|---|---|---|
| 3162 | | X | | X | | | | 2 |
| 2818 | X | X | X | X | X | X | X | 7 |
| 2754 | | | | X | | | | 1 |

TABLE 5 CONTINUED

| Molecular Weight of band (bp) | P1 | P3 | P4 | P5 | P6 | P7 | P8 | No of Parental Lines With the Band |
|---|---|---|---|---|---|---|---|---|
| 2750 | | X | | | X | | | 2 |
| 2455 | X | X | | X | X | X | | 5 |
| 2344 | | | | | | | X | 1 |

**TABLE 5 CONTINUED**

| Molecular Weight of band (bp) | P1 | P3 | P4 | P5 | P6 | P7 | P8 | No of Parental Lines With the Band |
|---|---|---|---|---|---|---|---|---|
| 2140 | X | X | X | X | X | X | X | 7 |
| 2042 | X | X | X | | X | | X | 5 |
| 1778 | X | X | X | X | X | X | X | 7 |
| 1700 | X | X | | X | X | X | | 5 |

TABLE 5 CONTINUED

| Molecular Weight of band (bp) | P1 | P3 | P4 | P5 | P6 | P7 | P8 | No of Parental Lines With the Band |
|---|---|---|---|---|---|---|---|---|
| 1660 | | | X | X | | | X | 3 |
| 1583 | | | | | X | | | 1 |
| 1514 | X | | X | | X | | | 3 |
| 1445 | X | X | X | X | X | X | X | 7 |
| Total No Of Bands In Each Parental Line | 8 | 9 | 7 | 9 | 10 | 6 | 7 | |

EP 0 337 625 A2

**TABLE 6**

| Band No | Lane Number | | | | | | | | | | | | | | | | | | | | Number of progeny inheriting band |
|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | |
| 5.11 | X | X | X | X | X | | X | X | X | | X | | X | X | X | | | | | | 12 |
| 5.10 | | X | X | X | X | X | X | X | X | X | X | X | | X | X | X | X | X | X | | 17 |
| 5.9 | | | | | | X | X | X | X | X | X | X | X | | | | X | X | X | | 11 |
| 5.8 | X | X | X | X | X | X | X | X | | X | X | X | X | X | X | X | X | X | X | X | 19 |
| | | | | | | | | | | | | | | | | | | | | | |
| 8.12 | X | X | X | X | X | X | X | X | | X | X | X | X | X | X | X | X | X | X | | 18 |
| 8.11 | X | X | X | X | X | X | X | | | X | | X | | X | X | X | X | | X | X | 14 |

## Claims

1. A process for obtaining hypervariable DNA probes useful in characterising plant material which comprises preparing purified DNA from a target plant species; cutting the purified DNA into fragments; cloning the fragments;

14

screening the cloned fragments with a Jeffreys probe and selecting one or more clones binding to said probe;
and multiplying such selected clones.

2. A process as claimed in claim 1 wherein the target plant species is a crop species.

3. A process as claimed in claim 2 in which the crop species is dicotyledonous.

4. A process as claimed in claim 2 in which the crop species is monocotyledonous.

5. A process as claimed in claim 4 in which the crop is a cereal.

6. A process as claimed in claim 5 in which the cereal is maize (Zea mays).

7. A process as claimed in claim 5 in which the cereal is rice (Oryza sativa).

8. A process as claimed in any of claims 1-7 wherein the Jeffreys probe is 33.6 or 33.15.

9. Hypervariable DNA clones useful in the characterisation of plant material and obtained by a process claimed in any of claims 1-8.

10. Hypervariable DNA clones useful in the characterisation of plant material having the repeat tandem sequence (AGAGGGCGGCGG).

11. Hypervariable DNA clones useful in the characterisation of plant material and having repeat tandem sequences at least 70% homologous with the repeat tandem sequences of the clones claimed in claim 10.

12. Process for characterising plant material which comprises preparing purified DNA from test plant material:
cutting the purified DNA with a selected restriction enzyme;
separating the cut DNA into an array of fragments by a standard separation technique;
probing the array of fragments with a hypervariable probe claimed in any of claims 9-11 and 16;
visualising the probe-fragment hybridisations and comparing the pattern so obtained with a corresponding pattern obtained from characterised plant material using the same restriction enzyme and the same probe under the same conditions.

13. Process as claimed in claim 12 wherein the hypervariable probe is derived from the same plant genus or species as the test plant material.

14. Process as claimed in claim 13 in which the plant species is Zea mays.

15. Process as claimed in claim 13 in which the plant species is Oryza sativa.

16. A process for obtaining hypervariable DNA probes useful in characterising plant material which comprises:
preparing purified DNA from a target plant species;
cutting the purified DNA into fragments;
cloning the fragments;
screening the cloned fragments with a hypervariable probe claimed in any of claims 9-11 and selecting one or more clones binding to said probe; and multiplying such selected clones.

17. A process claimed in claim 16 wherein the hypervariable DNA probe obtained is locus-specific.

# FIG. 1.

# FIG. 2.

EP 0 337 625 A2

# FIG. 3.

a b c d e f g h i j k l m n o p

# FIG. 4.

# FIG. 5.

a  b  c  d  e  f  g  h

# FIG. 6.

# FIG. 7.

FIG.8.

```
          10        20        30        40        50        60
GGATCCTTAA GGGTTATGTA CGGAACCATT CACACCCCGA GGGGAGTATC ATCGAGAGTT
          70        80        90       100       110       120
ACACCACCGA AGAGGTCATC GATTTTTGTG TGGACTAGAT GTCAGAAACA TCTTCAATTG
         130       140       150       160       170       180
GATTACCACG ATCTCATCAT GAAGGGAGGC TTGATGGTGT TGGTACTGTT GGAAGAAAGA
         190       200       210       220       230       240
CTGTTAGGTT GGATCGGAAA GGTGTACGAT AAAGCTCATT TCACGGTACC GCAGCATATG
         250       260       270       280       290       300
ACTGAGGTGG TGCCGTATGT TGACGAACAC CTTGCAGTTC TCCGACAGGA GAACCCAGAC
         310       320       330       340       350       360
CGATTAGAGA GTTGGGTCAG AAATAAGCAC ATGTCTTCTT TCAACGAGTG GCTGAAGAAC
         370       380       390       400       410       420
CGAATTGCTA GGTTGCAGAA CTTGTCTAGT AAAACACTTC AGTGGTTGTC ACAGGTCCTG
         430       440       450       460       470       480
AATGGAGTGT CACCACCTGG CAAGGATATG ACATAAATGG ATACATCTTT CACACGGTAA
         490       500       510       520       530       540
AGCAAGACAG CAAATGCACA GTGCACAACA GTGGGTTACG CATCGAGGTT GCTAGTGACG
         550       560       570       580       590       600
GTGGTCGTCG TGATAAATAC TATGGTAGAG TTGAGCAAAT ATTGGAGCTA GATTACTTGA
         610       620       630       640       650       660
AGTTCAAAGT CTCGTTGTTT CGTTGTCGAT AGGTCGATCT TCGCAATGTA AAAGTTGACA
         670       680       690       700       710       720
ATGAAGGTTT CACCACTGTC AACTTGGCTA ACAATGCGTA CAAGGATGAA CCGTTCGTTC
         730       740       750       760       770       780
TCGCCAAACA AGTTGTTCAA GTGTTCTACA TAGTTGACCC GTGTAACAAG AAACTACATG
         790       800       810       820       830       840
TTGTTCGTGA AGGGAAAAGG AGAATTGTTG GATTGGACAA TATTGCAGAC GAGGATGATT
         850       860       870       880       890       900
ACAACCAGCA CGTCCACGGC ATAGGTCAAG AAATACCTCT AGAAGAGGAG GAAGAAGAAG
         910       920       930       940       950       960
ATGACGTTCA ATATGCACGC ATCGACCATG AGGAAGGATT ATTTTTGTAA TTTATGTAAG
         970       980       990      1000      1010      1020
TAATTTATGT CTACATGTTT GTTATCTGTA TGACTCTAAT ATCTTCACAA TGATCAATAG
        1030      1040      1050      1060      1070      1080
TATATTGCTT ATGAAATAGT CAAATAAAAT AATTAAGTGA AGCACCAACT AGAAGTGAAA
        1090      1100      1110      1120      1130      1140
TAAAATAATT AAGCAAGTAT AACCATTGGA AATAAAATAA TAAAATTAGT AAAATAATGA
        1150      1160      1170      1180      1190      1200
AGTGCAATTA CTACTGTTAG TGAAATAAAA TAATTAAGTA TAACAAAAAT ATGTAGTGCA
        1210      1220
TGTGGAAATA TGTTAAGGAA AATAAAA--UNSEQUENCED GAP OF APPROX. 400bp--
          10        20        30        40        50        60
TAGGTTGGAC CTCTTCTAGA TCGATCCAGA TTCGGCACCC ACTCTCACTC ACTCACTCTC
          70        80        90       100       110       120
TCGACCGCCG CCGTCCTCTC CGCCGCCATC TCCACCCTCG CCGCCTCGAC CTCTCCGCCG
         130       140       150       160       170       180
CCCTCTCCGC TGCCATCTCC GCCGCCCTCT CTGCCGCCAT CTCCGCCCTA TCCGCCGCCC
         190       200       210       220       230       240
TCTCCACCGC CGCCTCGACC TCTCCACCGC CGCCCTCGCC GCCTCCCTCT CCACCGCCGC
         250       260       270       280       290       300
CGCCTCACTC ACTCTCTCGA CCGCCGCCGT CCTCTCCGCC GTCCGTCCCC GACCTCCCGA
         310
CACCGGGATC C
```

## FIG. 9.

```
          10         20         30         40         50         60
GGATCCTTAA GGGTTATGTA CGGAACCGTT CACACCCCGA GGGGAGTATC ATCGAGAGTT
          70         80         90        100        110        120
ACACCACCGA AGAGGCCATC GAATTTTGTG TGGACTACAT GTCCGAAACA TCTTCAATCG
         130        140        150        160        170        180
GATTACCACG ATCTCATCAC GAAGGGAGGC TTGACGGTGT TGGTACTGTT GGAAGAAAGA
         190        200        210        220        230        240
CTATTAGGTT GGATCGGAAA GGTGTACGAT AAAGCTCATT TCACGGTACT ACAGCATATG
         250        260        270        280        290        300
ACTGAGGTGG TGCCGTACGT TGACGAACAC CTTGCAGTTA TCCGACAAGA GAACCCAGGC
         310        320        330        340        350        360
CGATCAGAGA GTTGGGTCAG GAATAAGCAC ATGTCTTCTT TCAACGAGTG GCTGAAGAAC
         370        380        390        400        410        420
CGAATTGCCA GGTTGCAGAA CTTGTCTAGT GAAACACTTC AGTGGTTGTC ACAGGGTCCT
         430        440        450        460        470        480
GAATGGAGTG CCACCACCTG GCAAGATATG ACATAAATGG ATACACCTTT CACACGGTAA
         490        500        510        520        530        540
AGCAAGACAG CAAATACACA GTGCAGAACA GTGGGTTACG CATCGAGGCT GCTAGTGACG
         550        560        570        580        590        600
GTGGTCGTCG TGATCAATAC TATGGTAAAG TTGAGCAAAT ATTCGAGCTA GATTACTTGA
         610        620        630        640        650        660
AGTTCAAAGT CCCGTTGTTT CATTGTCGAT GGGTCGATCT TCGCAATGTA AAAGTTGACA
         670        680        690        700        710        720
ATGAAGGTTT CACCACTGTC AACTTGGCTA ACAACGACGT ACAAGGATGA ACCGTTCGTT
         730        740        750        760        770        780
CTCGCCAAAT AATTAAGTAT AAAAAAAATAT GTAGTGTATG TTAAAATATG TTAAGGAAAA
         790        800        810        820        830        840
TAAAAGAACT AAGTGAAATA AAATAAAATA AAATTGCTCT AGGCAAACTC ATCTGTGACG
         850        860        870        880        890        900
GGCTCTACCT GTAGAGCTTG CACAGGTGAC CTCACTGTGA CGGCCCAGAG TGTTGGGCAC
         910        920        930        940        950        960
TCACAGGTGG TCTTACCTGT GACGGCTCCA CGGTTGGCTG CAGCCACGGT GAGGCCACCT
         970        980        990       1000       1010       1020
GTGACGGCCA CATAGTTGGC GCCCGTCACA GGTGAGTTTG ACTAGGGTTG ACCTAGGGTT
        1030       1040       1050       1060       1070       1080
GGACATCTTC TAGATCGATC CAGATCCGGC ACCCACTCTC ACTCACTCAC TCTCTCGACC
        1090       1100       1110       1120       1130       1140
GCCGCCTCAC CTCTCTCTCT CTCGATCCAG ACCCTCCTCG CCGCCGCCTC GACCTCTCCA
        1150       1160       1170       1180       1190       1200
CCTCCCTCCT CGCCGCCGCC CTCTCCACCG CCATCTCCAC GTCGCCGCCT CAACCTCTCC
        1210       1220       1230       1240       1250       1260
TCCGCCCTCT CCGCCGCCAT CTCCGCTGCC CTCTCTGCCG CCATCTTCGC CGTCCTCTCC
        1270       1280       1290       1300       1310       1320
ACTGCCATCT TCACGTCGCT GCCTCAACCT CTCCTCCTCC CTCTCTGCCG CCATCTCCGC
        1330       1340       1350       1360       1370       1380
TGCCCTCTCT GCCGCCATCT CCGCCCTCTC CACCGCCGCC TCCGCCGCCG CCCTCACCGC
        1390       1400       1410       1420       1430       1440
CGGTCGTCCT CTCCGCCGTC CGTCCATCGA CTTCCCCGAC CTCCTCGACC CTGACCTCCC
        1450
GACGCCGGGA TCC
```

# FIG. 10.

FIG. 11.

EP 0 337 625 A2

```
                       10         20         30         40         50
MAIZE O.R.F.-SLRVMYGTVH TPRGVSSRVT PPKRPSNFVW TTCPKHLQSD YHDLITKGGL
             SLRVMYGT H TPRGVSSRVT PPKR S FVW T C KHLQ D YHDLI KGGL
RICE  O.R.F.-SLRVMYGTIH TPRGVSSRVT PPKRSSIFVW TRCQKHLQLD YHDLIMKGGL


                       60         70         80         90        100
MAIZE O.R.F.-TVLVLLEERL LGWIGKVYDK AHFTVLQHMT EVVPYVDEHL AVIRQENPGR
             VLVLLEERL LGWIGKVYDK AHFTV QHMT EVVPYVDEHL AVIRQENP R
RICE  O.R.F.-MVLVLLEERL LGWIGKVYDK AHFTVPQHMT EVVPYVDEHL AVLRQENPDR


                      110        120        130        140        150
MAIZE O.R.F.-SESWVRNKHM SSFNEWLKNR IARLQNLSSE TLQWLSQGPE WSATTWQDMT
             ESWVRNKHM SSFNEWLKNR IARLQNLSS  TLQWLSQ            DMT
RICE  O.R.F.-LESWVRNKHM SSFNEWLKNR IARLQNLSSK TLQWLSQVLN GVSPPGKDMT
```

# FIG. 12.

# FIG. 13.

A

B

# FIG. 14.

# FIG. 15.

# FIG. 16.